# EUROPEAN PATENT SPECIFICATION

(11) **EP 3 197 285 B1**
(45) Date of publication and mention of the grant of the patent: **06.09.2023**
(21) Application number: 15778044.6
(22) Date of filing: 23.09.2015
(51) Int. Cl.: A23D 9/00, A23D 9/02, C11B 1/06, C11B 3/00, C11B 3/04, C11B 7/00, C09D 15/00, C09D 191/00, B27K 3/08, B27K 3/15, C08L 91/00, A61L 2/00, A61L 9/00, B27K 3/00

(54) **SEQUENTIAL METHOD FOR PRODUCING PURIFIED, COLD-PRESSED LINSEED OIL WITH ENHANCED WOOD PENETRATION PROPERTIES**
SEQUENTIELLES VERFAHREN ZUR HERSTELLUNG VON GEREINIGTEM, KALTGEPRESSTEM LEINÖL MIT VERBESSERTEN HOLZPENETRATIONSEIGENSCHAFTEN
PROCÉDÉ SÉQUENTIEL DE PRODUCTION D'HUILE DE LIN PURIFIÉE, PRESSÉE À FROID PRÉSENTANT DES PROPRIÉTÉS DE PÉNÉTRATION DANS LE BOIS AMÉLIORÉES

(30) Priority: 23.09.2014 DK 201470586
(43) Date of publication of application: 02.08.2017
(73) Proprietor: SAGA Wood Holding AS, 3515 Hønefoss (NO)
(72) Inventor: KRISTENSEN, Lars Højmann, DK-9200 Aalborg SV (DK)
(74) Representative: Patrade A/S
(86) International application number: PCT/IB2015/057323
(87) International publication number: WO 2016/046760

(56) References cited:
- WO-A1-01/25376
- WO-A1-2006/135858
- CN-A- 103 045 356
- GB-A- 1 512 549
- US-A- 2 564 407

## Description

### Field of the Invention

The present invention relates to a method for production and purification of linseed oil. Finally, use of a linseed oil based impregnating agent for impregnation of wood is also disclosed.

### Background of the Invention

It is generally known to impregnate wood and that linseed oil with certain additives may be used as an impregnating agent.

From JP 2001 049289 a refined linseed oil and fat composition is known. This oil and fat composition is used as an additive to paints and inks, especially paints and inks used or exposed to high temperatures. The linseed oil and fat composition is treated with the aim of providing a high temperature stabilizing component in the paint or ink, such that damage to the paint/ink is minimized when exposed to high temperatures. The oil and fat composition is treated to a steam treatment in order to stabilized the oil and fat composition prior to being mixed with the paint or ink.

From GB 1 512 549 is a wood impregnating process known. In this process wood is introduced into a vacuum chamber wherein a solvent is present. Due to the vacuum and heating the solvent will replace moisture. In a following process step the solvent is replaced by an oil. The process requires specific relationships between the boiling points of the solvents and the oils. Furthermore there is no indication of using a refined linseed oil.

From CN 103 045 356 a refined linseed oil is known, for food or medical purposes. The oil is cold pressed, then treated with an acid, neutralized and undergoes a winterization step.

From WO 01/25376 a refined linseed oil is known, for use in the treatment of wood. The oil is cold pressed, then treated with an acid, neutralized and washed with water.

From US 2 564 407 a refined linseed oil is known, for use in paints or varnishes. The oil is extracted, winterized, treated with an acid and bleached.

### Object of the Invention

The present invention takes this knowledge a step further by presenting a method for production and purification of linseed oil for deep-penetration treatment of wood, incorporating extracting linseed oil by a cold pressing oil extraction process followed by sequential purification steps, performed under a Nitrogen blanket, said sequential purification steps involving aqueous acid mediated degumming followed by neutralization and refrigeration (winterization) of said linseed oil in order to precipitate and remove waxy contaminants and then blending the resulting linseed oil with mineral oil, in proportions ranging from mixtures of 95% linseed oil to 5% mineral oil to mixtures of 5% linseed oil to 95% mineral oil.

### Description of the Invention

The method is particular in that it produces very pure linseed oil.

The oil is extracted by a cold pressing oil extraction process.

The oil is of such quality and has such superior properties that it can penetrate wood to unexpectedly "great depths" (for example 150 mms when applied in a vacuum - pressure impregnation schedule, using pine as the substrate), and also / especially when blended with selected mineral oils.

To produce the oil of such quality it is necessary to process linseed in a cold-pressing regime, in that the oil is extracted using cold-pressing of the seeds rather than via application of a solvent extractant. Cold-pressing of the raw oil is carried out using known methodology. Although it is preferred to use cold pressing when extracting the oil, other methods of obtaining the linseed oil are also applicable. Other methods of obtaining linseed oil may for example be warm pressing. Through this process more impurities are suspended in the oil, which in turn requires extra cleaning/purification, thereby prolonging and making the method according to the invention more expensive.

This cold pressing yields typically in the range of 20-25% oil by mass from the seeds.

In a further preferred embodiment of the invention the method comprises the following process steps wherein:
a. the extracted oil is placed in a tank;
b. a solution of diluted sulphuric acid in mineral water containing between 0.1% and 3% sulphuric acid by weight is prepared in a second tank;
c. the liquids prepared in step a. and b. are then blended together in a single tank, with stirring at a temperature between 18°C and 32°C where the proportions of the liquids is 60% to 85% oil to 40% to 15% dilute sulphuric acid by volume;
d. the liquid mixture of step c. is kept under a flow or "blanket" of N2, to prevent oxidation, and the temperature is increased to between 40°C and 65°C;
e. the process is run with continual stirring under the N2 f flow or blanket for 5-7 hours, after which time the stirring is stopped;
f. the mix is left for 24 hours to separate into oil and aqueous phases after which the oil phase is then decanted from aqueous phase;
g. the decanted/separated oil is mixed and washed with demineralized water, in a new tank, and stirred for a further 2 hours under an N2 flow or blanket, during which period the water pH is continuously monitored to ensure removal of excess acid from oil;
h. the washed oil is cooled in refrigerated tanks and held at 4 - 5 °C for at least 20 hours, during which period waxy deposits crystallize / solidify and fall to the bottom of the tank;
i. the oil is then gently decanted from the top of the tank.

Cold pressed oil contains a number of impurities such as seed particles, phosphatides, carbohydrates including polysaccharides, proteins, pigments, waxes / paraffins, even pesticide residues. The oil needs further refining prior to use as a technical carrier liquid. The methods of preparing the oil further have been developed in a sequence and form the basis of a new processing regime for highly pure linseed oil suitable for penetrative wood treatment.

The following specific methods for cleaning the cold pressed oil have been developed:
Degumming:
The newly extracted cold-pressed oil is placed in a tank. A solution of dilute sulphuric acid in mineral water (containing between 0.3% and 1% sulphuric acid by weight) is prepared in a second tank.

The liquids are then blended together in a single tank, with stirring at a temperature of 25°C. This is in the proportions 75% oil, 25% dilute sulphuric acid (by volume). The blend is kept under a flow or "blanket" of N2, to prevent oxidation, and the temperature is increased to 50°C. The process is run with continual stirring (under N2) for 5-7 hours, after which time the stirring is stopped and the mix is left for 24 hours to separate into oil and aqueous phases. The oil phase is then decanted from aqueous phase.

The separated oil is next mixed with demineralized water, in a new tank, and stirred for a further 2 hours under N2. The water pH is continuously monitored to ensure removal of excess acid from oil. The procedure as described and obvious variants thereof lead to a degummed, cold-pressed linseed oil.

Further points to note with regard to the process are as follows:
The degumming process performed on the linseed oil will create residues, these can also be recycled in that the water is neutralized before it is then reused in the first step of the process.

A mixture of oil and water from the decanting step can be re-used in the first step of the process, or may be further decanted and extra oil recovered can be added to the main oil fraction.

Winterization:
This is a processing step designed to remove waxy residues from the washed and degummed oil. With regard to deep and effective penetration of wood in protective treatment regimes, waxy residues have negative impacts and their removal is a key step. In addition, they can interfere and cause problems with mixing of additives such as pigments and even other oils into the linseed oil phase.

The washed and degummed oil is cooled in refrigerated tanks and held at 4-5° C for at least 24 hours. During this period waxy deposits crystallize / solidify and fall to the bottom of the tank. The oil is then gently decanted from the top of the tank. This is a carefully applied step as the waxy remnants / precipitates are easily agitated and can be re-suspended by even gentle movement.

The oil is "in-process checked" for quality during the purification regime. This is as follows:
Degumming: Washed oil is mixed 50/50 with water and the mixture is left to stand for 24 hours, and is then examined. If properly degummed, a sharp dividing line should appear. If a whitish layer is formed at the interface, the degumming process needs to be repeated.

Any bacterial growth needs to be monitored using a system such as a Microsystems Count Combi. Any significant presence of phospholipids / polar lipids and water in the oil phase will lead to bacterial growth, and the testing can indicate this. If persistent, the degumming procedure needs to be repeated.

Winterisation: Remove a sample of oil after 24 hours of cooling, fill this in a clear glass beaker or measuring cylinder, and let this cool for a further 24 hours in a refrigerator. The bottom of the glass container is then examined and if a whitish sediment is observed, the winterization step needs to be repeated.

And finally in a further process step the linseed oil is blended with mineral oil, in proportions ranging from mixtures of 95 % linseed oil to 5% mineral oil to mixtures of 5% linseed oil to 95 % mineral oil.

The above processing gives rise to a purified, cold-pressed, linseed oil particularly suited to use in deep penetrative wood treatment.

Element 2: Principles of use of the new purified linseed oil in wood protective treatment

The new purified, cold-pressed linseed oil shows enhanced penetration of wood substrates, especially softwood (pine), fully penetrating to a depth of up to 150 mm into the wood. The oil is characterized as being substantially free of contaminants and waxes. Such contaminants are seed particles, phosphatides, carbohydrates including polysaccharides, proteins, pigments, and the above mentioned paraffins and waxes.

The new pure linseed oil has been successfully developed and used as the basis carrier solvent / liquid in a number of formulations designed for protective treatment of wood, in which deep penetration of the wood substrate is required.

The invention addresses using this oil, and in-blends with mineral oils, as basis "carrier" of wood treatment formulations for impregnation treatment of wood products. The invention also includes both vacuum and pressure impregnation regimes. A common further step optionally utilized is heating of the impregnated wood at temperatures ranging from 50°C to 100°C, to dry the wood, especially in cases wherein earlier aqueous treatments have been performed, and to help fix components of a treatment formulation within the wood matrix. The treatment regime can be carried out using an autoclave, optionally combined with vacuum treatment.

The purified linseed oil is used for the carrying of co-components such as pigments, preservatives / biocides and flame retardant compounds, optionally emulsified into the oil, into the substrate.

The new oil can also be used alone if only a penetrating hot (50°C - 100°C) or cold (ambient temperature) linseed oil treatment is required.

A further aspect of the invention is the combination or blending of the new purified linseed oil with selected mineral oils in order to improve properties of the oil and any resulting formulations based on the oil, in terms of wood substrate penetration and carrying of co-components such as pigments, biocides and flame retardant compounds, optionally emulsified into the oil, into the substrate.

The invention therefore encompasses the formulation of a number of blends of the "new linseed oil" as a direct treatment liquid for wood (examples) and as a base carrier for wood impregnation treatment. This can include vacuum and pressure impregnation regimes, as well as optionally a further step of heating the impregnated wood at temperatures ranging from 50°C to 100°C. The treatment regime can be carried out using an autoclave, optionally combined with vacuum treatment.

The invention therefore also further encompasses the formulation of a number of blends of the "new linseed oil" and mineral oils as a direct treatment liquid for wood (examples) and as a base carrier for wood impregnation treatment with flame-retardant salts / compounds, pigments, biocides. This often includes vacuum and pressure impregnation regimes, as well as optionally a further step of heating the impregnated wood at temperatures ranging from 50°C to 100°C. The treatment regime can be carried out using an autoclave, optionally combined with vacuum treatment.

The invention, via the beneficial properties of the cold-pressed, purified linseed oil and blends with mineral oils in formulations, particularly addresses the need to carry pigments and protective compounds such as flame-retardants and biocides, deep into the wood substrate and to bind these compounds into the wood at these penetration depths. Thus improving the level of treatment and protection over other conventional systems and the length of time that the protection and treatment is effective.

There is a particular benefit in using the new oil based treatment for application of flame retardant compounds, optionally as an oil-based "water in oil" emulsion, in that other treatments (such as surface application in a paint, by brushing, spraying or dipping, or by pressure treatment using an aqueous solution of a fire retardant salt or salt mix), can be limited to surface treatments alone, or minimal penetration of the wood. Alternatively, when salts such as ammonium phosphate are applied as aqueous solutions, these are easily leached from the wood. The correctly applied process based on the new purified linseed oil and its formulations leads to deep penetration of the flame retardant into the wood and fixing of the compounds, preventing or inhibiting pronounced leaching from the wood over time.

The hardening of the oil-mixture can be controlled by adding compounds. In order to accelerate the hardening siccatives (driers - such as Co, Mn, Pb, Zr or Fe) or by adding energy (UV light or heat) may be added, whereas in order to slow down the hardening antioxidants or anti-skin compounds may be added.

The autoclave, optionally combined with vacuum treatment typically comprises an oil containing vessel where all parameters (see method description elsewhere) are continuously monitored and adjusted. The vessel is arranged inside an enclosure where an under-pressure ("vacuum") is or can be applied. All parameters relating to level of under-pressure, time in treatment (wood above oil, in oil, with and/or without under-pressure etc.) are all carefully monitored, controlled and adjusted.

The basic linseed or mixed oil based treatment is preferably carried out in an autoclave in which the wood is optionally placed under vacuum. The characteristics of the finished treated wood are the imparting, via the treatment (using linseed oil and mixes of linseed oil and mineral oils as the oil phase), of selected properties, and addition of varying target, levels of purified linseed oil to the wood. Additives to the oil are selected (often in combination) to engender the following properties / effects on the treated wood, Examples of the additives chosen will be to create:
Wood protection
Fire protection
Marine Protection (protection against rot and fungus in both fresh and saltwater) Moisture stabilization (dimensional stabilization)
Accelerated graying
Defibering - such that the wood surface looks like a brushed surface

A further option is to produce a surface coating or treatment based on linseed oil. The linseed oil alone gives a "natural finish". However, by combining the linseed oil, which penetrates deeply into the wood, with natural pigments and pigment blends that can only partially penetrate or fix onto the surface, the process can be used to create exactly the color on the surface requested by the customer. It provides a number of options for choice of colour.

## Claims

1. A method for production and purification of linseed oil for deep-penetration treatment of wood, said method incorporating extracting linseed oil by a cold pressing oil extraction process followed by sequential purification steps, performed under a Nitrogen blanket, said sequential purification steps involving aqueous acid mediated degumming followed by neutralization and refrigeration (winterization) of said linseed oil in order to precipitate and remove waxy contaminants and wherein in a further method step the linseed oil is blended with mineral oil, in proportions ranging from mixtures of 95 % linseed oil to 5% mineral oil to mixtures of 5% linseed oil to 95 % mineral oil.

2. The method according to claim 1 wherein in further method steps_pigments are mixed into the linseed oil where the concentration of pigment range from 0.1% to 5% on a weight percent basis, and where the pigments are selected among iron oxide, titanium dioxide, carbon black and/or preservatives or biocides are mixed into the linseed oil where the biocide or preservative levels range from 0.02% to 3% on a weight percent basis, and where the preservatives or biocides are selected among propiconazole, IPBC, iron oxide, ammonia salts, borate salts, and/or creosote in environmentally friendly form and/or flame retardant salts and compounds are mixed or emulsified into the linseed oil where the flame retardant salt or compound levels range from 1% to 15% on a weight percent basis, and where the salts or compounds are selected among Ammonium phosphate, Borates, Aluminium Hydroxide, Magnesium Hydroxide, Organophosphates, phosphonates, phosphinates.

3. The method according to either claim 1 or 2 wherein:
a. the extracted oil is placed in a tank;
b. a solution of diluted sulphuric acid in mineral water containing between 0.1% and 3 % sulphuric acid by weight is prepared in second tank;
c. the liquids prepared in step a. and b. are then blended together in a single tank, with stirring at a temperature between 18°C and 32°C where the proportions of the liquids is 60% to 85% oil to ,40% to 15% dilute sulphuric acid by volume;
d. the liquid mixture of step c. is kept under a flow or "blanket" of N2, to prevent oxidation, and the temperature is increased to between 40°C and 65°C
e. the process is run with continual stirring under the N₂ flow or blanket for 5-7 hours, after which time the stirring is stopped and
f. the mix is left for 24 hours to separate into oil and aqueous phases after which the oil phase is then decanted from aqueous phase
g. the decanted/separated oil is mixed and washed with demineralized water, in a new tank, and stirred for a further 2 hours under an N2 flow or blanket, during which period the water pH is continuously monitored to ensure removal of excess acid from oil;
h. the washed oil is cooled in refrigerated tanks and held at 4 - 5 C for at least 20 hours, during which period waxy deposits crystallise / solidify and fall to the bottom of the tank;
i. the oil is then gently decanted from the top of the tank;
j. and wherein in a further process step the linseed oil is blended with mineral oil, in proportions ranging from mixtures of 95 % linseed oil to 5% mineral oil to mixtures of 5% linseed oil to 95 % mineral oil.

4. Use of a cold pressed linseed oil manufactured and purified according to a method according to any of claims 1 to 3 involving the following method steps extracting linseed oil by a cold pressing oil extraction process followed by sequential purification steps, performed under a Nitrogen blanket, said sequential purification steps involving aqueous acid mediated degumming followed by neutralization and refrigeration (winterization) of said linseed oil in order to precipitate and remove waxy contaminants, followed by a further method step wherein the linseed oil is blended with mineral oil, in proportions ranging from mixtures of 95 % linseed oil to 5% mineral oil to mixtures of 5% linseed oil to 95 % mineral oil, and_where the cold pressed linseed oil is used for treatment of wood, especially softwoods such as pines and spruce, where said treatment includes sequential vacuum and pressure impregnation to help oil penetration into the wood substrate, where this may be followed by heating the treated wood to temperatures ranging from 50°C to 100°C and where the treatment is optionally carried out using a "vacuum autoclave" or alternatively where the treatment of wood is by dip- ping brush or spray application.

## Patentansprüche

1. Verfahren zur Herstellung und Reinigung von Leinöl zur Tiefenpenetrationsbehandlung von Holz, wobei das Verfahren Extrahieren von Leinöl durch einen Kaltpress-Ölextraktionsprozess gefolgt von sequentiellen Reinigungsschritten einbezieht, die unter einer Stickstoffdecke durchgeführt werden, wobei die sequentiellen Reinigungsschritte durch wässrige Säure vermitteltes Degummieren gefolgt von Neutralisierung und Kühlung (Winterisierung) des Leinöls einschließen, um wachsartige Verunreinigungen auszufällen und zu entfernen, und wobei in einem weiteren Verfahrensschritt das Leinöl mit Mineralöl in Verhältnissen vermischt wird, die von Gemischen aus 95 % Leinöl zu 5 % Mineralöl bis Gemischen aus 5 % Leinöl zu 95 % Mineralöl reichen.

2. Verfahren nach Anspruch 1, wobei in weiteren Verfahrensschritten Pigmente in das Leinöl gemischt werden, wobei die Pigmentkonzentration von 0,1 % bis 5 % auf Gewichtsprozentbasis reicht und wobei die Pigmente aus Eisenoxid, Titandioxid, Ruß ausgewählt sind, und/oder Konservierungsstoffe oder Biozide in das Leinöl gemischt werden, wobei die Spiegel an Bioziden oder Konservierungsstoffen von 0,02 % bis 3 % auf Gewichtsprozentbasis reichen und wobei die Konservierungsstoffe oder Biozide aus Propiconazol, IPBC, Eisenoxid, Ammoniumsalzen, Boratsalzen und/oder Kreosot in umweltfreundlicher Form ausgewählt sind, und/oder flammhemmende Salze und Verbindungen in das Leinöl gemischt oder emulgiert werden, wobei die Spiegel an flammhemmenden Salzen oder Verbindungen von 1 % bis 15 % auf Gewichtsprozentbasis reichen und wobei die Salze oder Verbindungen aus Ammoniumphosphat, Boraten, Aluminiumhydroxid, Magnesiumhydroxid, Organophosphaten, Phosphonaten, Phosphinaten ausgewählt sind.

3. Verfahren nach entweder Anspruch 1 oder 2, wobei:
a. das extrahierte Öl in einen Tank gegeben wird;
b. eine Lösung von verdünnter Schwefelsäure in Mineralwasser, die zwischen 0,1 Gewichts-% und 3 Gewichts-% Schwefelsäure enthält, in einem zweiten Tank zubereitet wird;
c. die in Schritt a. und b. zubereiteten Flüssigkeiten dann in einem einzigen Tank unter Rühren bei einer Temperatur zwischen 18 °C und 32 °C vermischt werden, wobei die Verhältnisse der Flüssigkeiten 60 Volumen-% bis 85 Volumen-% Öl zu 40 Volumen-% bis 15 Volumen-% verdünnter Schwefelsäure betragen;
d. das flüssige Gemisch aus Schritt c. unter einer Strömung oder "Decke" von N2 gehalten wird, um Oxidation zu verhindern, und die Temperatur auf zwischen 40 °C und 65 °C erhöht wird
e. der Prozess unter kontinuierlichem Rühren unter der N₂-Strömung oder -Decke 5-7 Stunden laufen gelassen wird, wonach das Rühren gestoppt wird, und
f. das Gemisch 24 Stunden stehen gelassen wird, um sich in eine Öl- und eine Wasserphase zu trennen, wonach die Ölphase dann von der Wasserphase abdekantiert wird
g. das abdekantierte/abgetrennte Öl in einem neuen Tank mit entmineralisiertem Wasser gemischt und gewaschen und weitere 2 Stunden unter einer N2-Strömung oder -Decke gerührt wird, wobei während dieses Zeitraums der pH-Wert des Wassers kontinuierlich überwacht wird, um die Entfernung von überschüssiger Säure aus dem Öl sicherzustellen;
h. das gewaschene Öl in Kühltanks gekühlt wird und mindestens 20 Stunden bei 4-5 °C gehalten wird, wobei während dieses Zeitraums wachsartige Ablagerungen kristallisieren/erstarren und auf den Boden des Tanks fallen;
i. das Öl dann vorsichtig von der Oberseite des Tanks abdekantiert wird;
j. und wobei in einem weiteren Prozessschritt das Leinöl mit Mineralöl in Verhältnissen vermischt wird, die von Gemischen aus 95 % Leinöl zu 5 % Mineralöl bis Gemischen aus 5 % Leinöl zu 95 % Mineralöl reichen.

4. Verwendung eines kaltgepressten Leinöls, gefertigt und gereinigt gemäß einem Verfahren nach einem der Ansprüche 1 bis 3, das die folgenden Verfahrensschritte einschließt: Extrahieren von Leinöl durch einen Kaltpress-Ölextraktionsprozess gefolgt von sequentiellen Reinigungsschritten, die unter einer Stickstoffdecke durchgeführt werden, wobei die sequentiellen Reinigungsschritte durch wässrige Säure vermitteltes Degummieren gefolgt von Neutralisierung und Kühlung (Winterisierung) des Leinöls einschließen, um wachsartige Verunreinigungen auszufällen und zu entfernen, gefolgt von einem weiteren Verfahrensschritt, bei dem das Leinöl mit Mineralöl in Verhältnissen vermischt wird, die von Gemischen aus 95 % Leinöl zu 5 % Mineralöl bis Gemischen aus 5 % Leinöl zu 95 % Mineralöl reichen, und wobei das kaltgepresste Leinöl zur Behandlung von Holz, insbesondere Weichhölzern wie etwa Kiefern und Fichte, verwendet wird, wobei die Behandlung sequentielle Vakuum- und Druckimprägnierung beinhaltet, um bei der Ölpenetration in das Holzsubstrat zu helfen, wobei darauf Erwärmen des behandelten Holzes auf Temperaturen folgen kann, die von 50 °C bis 100 °C reichen, und wobei die Behandlung optional unter Verwendung eines "Vakuumautoklavs" ausgeführt wird oder wobei alternativ die Behandlung von Holz durch Tauchpinsel oder Sprühauftrag erfolgt.

## Revendications

1. Procédé de production et de purification d'huile de lin pour le traitement de pénétration profonde du bois, ledit procédé incorporant l'extraction de l'huile de lin par un procédé d'extraction d'huile par pressage à froid suivi d'étapes de purification séquentielles, effectuées sous une couverture d'azote, lesdites étapes de purification séquentielles impliquant un dégommage aqueux à l'acide suivi d'une neutralisation et d'une réfrigération (hivérisation) de ladite huile de lin afin de précipiter et d'éliminer les contaminants cireux et dans lequel, dans une autre étape de procédé, l'huile de lin est mélangée avec une huile minérale, dans des proportions s'échelonnant de mélanges de 95 % d'huile de lin à 5 % d'huile minérale à des mélanges de 5 % d'huile de lin à 95 % d'huile minérale.

2. Procédé selon la revendication 1, dans lequel, dans d'autres étapes de procédé, des pigments sont mélangés dans l'huile de lin où la concentration de pigment s'échelonne de 0,1 % et 5 % sur une base de pourcentage en poids, et où les pigments sont choisis parmi l'oxyde de fer, le dioxyde de titane, le noir de carbone et/ou des conservateurs ou des biocides sont mélangés dans l'huile de lin où les niveaux de biocide ou de conservateur s'échelonnent de 0,02 % à 3 % sur une base de pourcentage en poids, et où les conservateurs ou les biocides sont choisis parmi le propiconazole, l'IPBC, l'oxyde de fer, les sels d'ammoniaque, les sels de borate et/ou la créosote sous une forme respectueuse de l'environnement et/ou des sels et composés ignifuges sont mélangés ou émulsifiés dans l'huile de lin où les niveaux de sel ou de composé ignifuge s'échelonnent de 1 % à 15 % sur une base de pourcentage en poids, et où les sels ou composés sont choisis parmi le phosphate d'ammonium, les borates, l'hydroxyde d'aluminium, l'hydroxyde de magnésium, les organophosphates, les phosphonates, les phosphinates.

3. Procédé selon l'une des revendications 1 ou 2, dans lequel :
a. l'huile extraite est placée dans une cuve ;
b. une solution d'acide sulfurique dilué dans de l'eau minérale contenant entre 0,1 % et 3 % en poids d'acide sulfurique est préparée dans une seconde cuve ;
c. les liquides préparés aux étapes a. et b. sont ensuite mélangés dans une seule cuve, sous agitation à une température comprise entre 18 °C et 32 °C où les proportions des liquides sont de 60 % à 85 % d'huile pour 40 % à 15 % d'acide sulfurique dilué en volume ;
d. le mélange de liquides de l'étape c. est maintenu sous un flux ou « couverture » de N2, pour empêcher l'oxydation, et la température est élevée à une température comprise entre 40 °C et 65 °C
e. le procédé est exécuté avec une agitation continue sous le flux ou la couverture de N₂ pendant 5 à 7 heures, après quoi l'agitation est arrêtée et
f. le mélange est laissé pendant 24 heures pour se séparer en phases huileuse et aqueuse, après quoi la phase huileuse est ensuite décantée de la phase aqueuse
g. l'huile décantée/séparée est mélangée et lavée avec de l'eau déminéralisée, dans une nouvelle cuve, et agitée pendant 2 heures supplémentaires sous un flux ou une couverture de N2, période pendant laquelle le pH de l'eau est surveillé en continu pour assurer l'élimination de l'excès d'acide de l'huile ;
h. l'huile lavée est refroidie dans des cuves réfrigérées et maintenue à 4 - 5 °C pendant au moins 20 heures, période pendant laquelle les dépôts cireux se cristallisent/se solidifient et tombent au fond de la cuve ;
i. l'huile est ensuite décantée doucement du haut de la cuve ;
j. et dans lequel, dans une autre étape de procédé, l'huile de lin est mélangée avec une huile minérale, dans des proportions s'échelonnant de mélanges de 95 % d'huile de lin à 5 % d'huile minérale à des mélanges de 5 % d'huile de lin à 95 % d'huile minérale.

4. Utilisation d'une huile de lin pressée à froid fabriquée et purifiée par un procédé selon l'une quelconque des revendications 1 à 3 impliquant les étapes de procédé suivantes d'extraction de l'huile de lin par un procédé d'extraction d'huile par pressage à froid suivies d'étapes de purification séquentielles, réalisées sous une couverture d'azote, lesdites étapes de purification séquentielles impliquant un dégommage aqueux à l'acide suivi d'une neutralisation et d'une réfrigération (hivérisation) de ladite huile de lin afin de précipiter et d'éliminer les contaminants cireux, suivies d'une autre étape de procédé dans laquelle l'huile de lin est mélangée avec une huile minérale, dans des proportions s'échelonnant de mélanges de 95 % d'huile de lin à 5 % d'huile minérale à des mélanges de 5 % d'huile de lin à 95 % d'huile minérale, et où l'huile de lin pressée à froid est utilisée pour le traitement du bois, en particulier des bois tendres tels que les pins et l'épicéa, où ledit traitement comporte une imprégnation séquentielle sous vide et sous pression pour faciliter la pénétration de l'huile dans le substrat en bois, où celui-ci peut être suivi d'un chauffage du bois traité à des températures s'échelonnant de 50 °C à 100 °C et où le traitement est éventuellement effectué à l'aide d'un « autoclave sous vide » ou en variante où le traitement du bois se fait par trempage au pinceau ou par pulvérisation.
